# EUROPÄISCHE PATENTSCHRIFT

(11) **EP 1 455 824 B1**
(45) Veröffentlichungstag und Bekanntmachung des Hinweises auf die Patenterteilung: **06.08.2008**
(21) Anmeldenummer: 02792797.9
(22) Anmeldetag: 25.11.2002
(51) Int. Cl.: A61K 39/395, A61K 47/18, A61K 47/26, A61K 47/34, A61P 35/00

(54) **LYOPHILISIERTE ZUBEREITUNG ENTHALTEND ANTIKÖRPER GEGEN DEN EGF-REZEPTOR**
LYOPHILIZED PREPARATION CONTAINING ANTIBODIES TO THE EGF RECEPTOR
PREPARATION LYOPHILISEE RENFERMANT DES ANTICORPS LUTTANT CONTRE LE RECEPTEUR DU FACTEUR DE CROISSANCE EPIDERMIQUE EGF

(30) Priorität: 21.12.2001 DE 10163459
(43) Veröffentlichungstag der Anmeldung: 15.09.2004
(73) Patentinhaber: Merck Patent GmbH, 64293 Darmstadt (DE)
(72) Erfinder: MAHLER, Hanns-Christian, 65205 Wiesbaden (DE); ZOBEL, Hans-Peter, 65439 Flörsheim (DE); MÜLLER, Robert, 64289 Darmstadt (DE); BACHMANN, Christiane, 66773 Gladbach (DE); HAAS, Udo, 64293 Darmstadt (DE); KRÜGER, Ludwig, 63741 Aschaffenburg (DE); MARTINI-MARR, Ulrike, D- 79591 Eimeldingen (DE)
(86) Internationale Anmeldenummer: PCT/EP2002/013223
(87) Internationale Veröffentlichungsnummer: WO 2003/053465

(56) Entgegenhaltungen:
- WO-A-98/22136
- A.A. MORALES MORALES ET AL.: "Freeze-dried formulation for direct 99mTc-labeling ior-egf/r3 MAb: Additives, biodistribution, and stability." NUCLEAR MEDICINE & BIOLOGY, Bd. 26, Nr. 6, August 1999 (1999-08), Seiten 717-723, XP002252112
- J. BASELGA : "The EGFR as a target for anticancer therapy-focus on cetuximab" EUROPEAN JOURNAL OF CANCER, PERGAMON PRESS, OXFORD, GB, Bd. 37, Nr. Suppl. 4, September 2001 (2001-09), Seiten S16-S22, XP004307911 ISSN: 0959-8049
- H. BIER ET AL.: "Anti-(epidermal growth factor) receptor monoclonal antibodies for the induction of antibody-dependent cell-mediated cytotoxicity against squamous cell carcinoma lines of the head and neck." CANCER IMMUNOLOGY IMMUNOTHERAPY, Bd. 46, Nr. 3, Mai 1998 (1998-05), Seiten 167-173, XP002252113 in der Anmeldung erwähnt
- LAI M.C.; TOPP E.M.: 'Solid-State Chemical Stability of Proteins and Peptides' JOURNAL OF PHARMACEUTICAL SCIENCES Bd. 88, Nr. 5, Mai 1999, Seiten 489 - 500

## Beschreibung

Die vorliegende Erfindung betrifft eine stabile lyophilisiert pharmazeutische Zubereitung enthaltend einen Antikörper, die gegen den Rezeptor des epidermalen Wachstumsfaktors (EGFR) gerichtet ist und deren Herstellung.

In verschiedenen in vitro und in vivo Studien konnte gezeigt werden, dass die Blockade des EGFR durch Antikörper auf unterschiedlichen Ebenen gegen Tumore wirkt, beispielsweise durch Hemmung der Krebszellproliferation, Verringerung der tumorvermittelten Angiogenese, Induktion der Krebszellapoptose und Verstärkung der toxischen Wirkungen der Strahlentherapie und der herkömmlichen Chemotherapie.

MAB c225 (Cetuximab) ist ein klinisch erprobter Antikörper, der an den EGF-Rezeptor bindet. Cetuximab^{®} ist ein chimärer Antikörper, dessen variable Regionen murinen und dessen konstante Regionen humanen Ursprungs sind, und wurde erstmals von Naramura et al., Cancer Immunol Immunotherapy 1993, 37: 343-349 sowie in WO 96/40210 A1 beschrieben.

MAB 425 ist ein ursprünglich muriner auf Tumor-Zellen überexprimierter Antikörper, der gegen den EGFR, insbesondere von A431 Carcinoma-Zellen, gerichtet ist. Seine humanisierten und chimären Formen sind z.B. aus EP 0 531 472 A1; Kettleborough et al., Protein Engineering 1991, 4: 773-783; Bier et al. , Cancer Chemother Pharmacol 2001, 47: 519-524; Bier et al., Cancer Immunol Immunother 1998, 46: 167-173 bekannt. EMD 72000 ist dabei ein in der klinischen Phase I/II befindlicher Antikörper (h425), dessen konstanter Bereich sich aus einer κ und einer humanen γ-1-Kette zusammen setzt.

Humane anti-EGFR-Antikörper können nach der XenoMouse-Technologie bereitgestellt werden, wie beschrieben in WO 91/10741 A1, WO 94/02602 A1, WO 96/33735 A1. Ein gemäß dieser Technologie hergestellter spezieller in der klinischen Erprobung befindlicher Antikörper ist ABX-EGF (Abgenix, Crit Rev Oncol Hematol 2001, 38: 17-23; Cancer Research 1999, 59: 1236-43).

Weitere gegen den EGFR gerichtete Antikörper sind z.B. beschrieben in EP 0 586 002 B1 sowie in J Natl Cancer Inst 1993, 85: 27-33 (MAB 528).

Wie andere Antikörper werden auch die EGFR-Antikörper zur therapeutischen Anwendung parenteral als Lösung appliziert. Ein besonderes Problem von Lösungen mit diesen Antikörpern ist deren Neigung zur Aggregation und zur Bildung von Proteinmultimeren. Im Falle reduzierbarer Multimere kann dies auf nicht beabsichtigte intermolekulare Disulfidbrückenbildung durch eine Interaktion zwischen sich annähernden Molekülteilen zurückgeführt werden. Auch kommen hydrophobe Wechselwirkungen und die damit verbundene Bildung nichtreduzierbarer Multimere in Betracht. Weiterhin kommt es zu Deamidierungsreaktionen, die nachfolgend zu Proteinabbaureaktionen führen. Die beschriebenen Denaturierungsreaktionen treten besonders bei Lagerung bei erhöhter Temperatur auf oder bei Scherbeanspruchung, wie sie beispielweise beim Transport auftritt. Insgesamt sind flüssige Zubereitungen daher als Arzneiform für die breite Anwendung weniger geeignet.

Ein gebräuchliches Verfahren zur Stabilisierung von Antikörpern ist die Gefriertrocknung von Lösungen, die Antikörper sowie Hilfsstoffe enthalten. Durch Entfernung des Wasser wird die Bildung von Zersetzungsprodukten und Aggregaten reduziert (Hsu et al., Dev. Biol. Stand. 1991, 74: 255-267 und Pikal et al., Dev. Biol. Stand. 1991, 74: 21-27).

WO 93/00807 A1 beschreibt lyophilisierte Zubereitungen von Proteinen, die zur Stabilisierung Polyethylenglycole und einen Zucker enthalten. Polyethylenglycole sind jedoch toxikologisch nicht unbedenklich und sollten daher in Arzneimitteln, insbesondere wenn sie zur parenteralen Verabreichung vorgesehen sind, wenn möglich vermieden werden.

WO 98/22136 A2 offenbart eine lyophilisierte Zubereitung enthaltend einen Antikörper, einen Zucker oder Aminozucker, eine Aminosäure und ein Tensid. Zwar wird die Zubereitung für Antikörper im allgemeinen beansprucht, als Ausführungsbeispiel offenbart sind jedoch nur Zubereitungen mit monoklonalen Antikörpern, die gegen den Hepatitis B-Virus (AK HBV) gerichtet sind, sowie jeweils eine Zubereitung mit einem Antikörper gegen L-Selektin (Anti-L-Selektin) und einem Antikörper gegen den Anti-L-Nerve-Growth-Factor-Rezeptor (Anti-L-NGFR). Während die Zubereitungen mit AK HBV und Anti-L-Selektin aus Lösungen mit einer Antikörperkonzentration von maximal 8 mg/ml bzw. 7 mg/ml hergestellt wurden, wurde die Zubereitung mit dem gegen den Wachstumsfaktor gerichteten Antikörper Anti-L-NGFR bei ansonsten identischer qualitativer und quantitativer Zusammensetzung der Hilfsstoffe aus einer Lösung mit nur 0,25 mg/ml Antikörper hergestellt. Obwohl die Zubereitung mit Anti-L-NGFR damit einen mehr als 20-fach geringeren Antikörperanteil enthält und daher auch eine entsprechend geringere Menge an Abbauprodukten zu erwarten ist, werden im Gegensatz zu den Zubereitungen mit den anderen Antikörpern keine Stabilitätsdaten offenbart.

Es war Aufgabe der vorliegenden Erfindung eine stabilisierte Zubereitung für die gegen den EGFR gerichtete Antikörper zur Verfügung zu stellen. Die Zubereitung sollte keine toxikologisch bedenklichen Hilfsstoffe enthalten, unter erhöhten Stressbedingungen, wie erhöhter Temperatur und Luftfeuchtigkeit, über längere Zeit stabil sein und mit einem wässrigen Lösungsmittel zu einer applikationsfertigen Lösung mit hohem Wirkstoffanteil rekonstituierbar sein.

Überraschenderweise konnte eine diesen Anforderungen entsprechende Zubereitung zur Verfügung gestellt werden, indem eine wässrige Lösung, die neben einem dieser anti-EGFR-Antikörper einen Zucker oder einem Aminozucker, eine Aminosäure und ein Tensid enthält, gefriergetrocknet wird. Gegenstand der vorliegenden Erfindung ist daher eine stabile lyophilisierte Zubereitung von mono- oder polyklonalen Antikörpern, enthaltend einen Zucker oder einen Aminozucker, eine Aminosäure und ein Tensid, **dadurch gekennzeichnet, dass** der Antikörper Cetuximab oder Matuzumab (EMO 72000) ist.

Die erfindungsgemäße Zubereitung ist physiologisch gut verträglich, leicht herstellbar, exakt dosierbar und über die Dauer der Lagerung und bei mehrfachen Einfrier- und Auftauvorgängen hinsichtlich Gehalt, Zersetzungsprodukten und Aggregaten stabil. Sie kann bei Kühlschranktemperatur (2-8°C) und bei Raumtemperatur (23-27°C, 60 % relative Luftfeuchtigkeit (r.F.)) über einen Zeitraum von mindestens drei Monaten bis zu einem Zeitraum von einem bis zwei Jahren stabil gelagert werden. Überraschenderweise ist die erfindungsgemäße Zubereitung auch bei höheren Temperaturen und Luftfeuchtigkeiten, beispielsweise bei einer Temperatur von 40°C und 75% relativer Luftfeuchtigkeit über den genannten Zeitraum stabil lagerbar.

Die lyophilisierte Zubereitung kann in einfacher Weise durch Zugabe eines wässrigen Lösungsmittels, beispielsweise Wasser für Injektionszwecke oder einer isotonischen wässrigen Lösung, zu einer applikationsfertigen Lösung rekonstituiert werden, die keine sichtbaren Partikeln aufweist. Die rekonstituierte Lösung ist über einen Zeitraum von etwa 5 Tagen stabil, wird aber besonders bevorzugt innerhalb von vier Stunden appliziert.

Vorteilhaft können aus der erfindungsgemäßen Zubereitung durch Rekonstitution mit wässrigen Lösungsmitteln antikörperhaltige Lösungen mit einem pH-Wert von 5 bis 8, bevorzugt mit einem pH-Wert von 6,0 bis 7,4, besonders bevorzugt mit einem pH-Wert von cirka 7,2 und einer Osmolalität von 250 bis 350 mOsmol/kg hergestellt werden. Die rekonstituierte Zubereitung kann somit weitgehend schmerzfrei intravenös, intraarteriell und auch subkutan direkt verabreicht werden. Daneben kann die Zubereitung auch Infusionslösungen, wie z.B. Glukoselösung, isotonische Kochsalzlösung oder Ringerlösung, die auch weitere Wirkstoffe enthalten können, zugesetzt werden, so dass auch größere.

### Wirkstoffmengen appliziert werden können

Gemäß einer bevorzugten Ausführungsform der Erfindung besteht die lyophilisierte pharmazeutische Zubereitung im wesentlichen aus einem Antikörper, einem Zucker oder Aminozucker, einer Aminosäure, einem Puffer und einem Tensid.

Die erfindungsgemäße Zubereitung ermöglicht die Herstellung von Antikörperlösungen, die in ihrer Konzentration den klinischen Erfordernissen angepasst sind. Bevorzugt sind Antikörperlösungen mit einer Antikörperkonzentration von ca. 0,5 bis 25 mg/ml, besonders bevorzugt sind 5 bis 20 mg/ml, ganz besonders bevorzugt sind 10 bis 15 mg/ml. Die erfindungsgemäße Zubereitung ermöglicht somit die Herstellung von applikationsfertigen Zubereitungen mit deutlich höherer Antikörperkonzentration als dies für die Zubereitungen von WO 98/22136 A2 beschrieben ist.

In der erfindungsgemäßen Zubereitung können als Zucker Mono-, Di- oder Trisaccharide eingesetzt werden. Beispielhaft genannt seien als Monosaccharide Glucose, Mannose, Galactose, Fructose und Sorbose, als Disaccharide Saccharose, Lactose, Maltose oder Trehalose und als Trisaccharid Raffinose. Bevorzugt sind Saccharose, Lactose, Maltose oder Trehalose enthalten, besonders bevorzugt ist Saccharose. Ebenso können Aminozucker enthalten sein, d.h. Monosaccharide, die anstelle einer Hydroxygruppe eine primäre, sekundäre oder tertiäre Amino- oder eine acylierte Aminogruppe (-NH-CO-R) besitzen. Erfindungsgemäß besonders bevorzugt sind hierbei Glucosamin, N-Methylglucosamin, Galactosamin und Neuraminsäure.

Der enthaltene Zucker/Aminozucker liegt in der erfindungsgemäßen Zubereitung in einer Menge vor, dass dieser nach Rekonstitution mit der vorgesehenen Volumen an Lösungsmittel in der erhaltenen Lösung in einer Konzentration von cirka 1 bis 200 mg/ml vorliegt. Bevorzugt liegt der Zucker in der rekonstituierten Lösung in einer Konzentration von 30 bis 65 mg/ml vor.

Als erfindungsgemäß verwendete Aminosäuren kommen basische Aminosäuren in Frage, wie beispielsweise Arginin, Histidin, Ornithin, Lysin u.a., wobei die Aminosäuren vorzugsweise in Form ihrer anorganischen Salze (vorteilhaft in Form der Salzsäuresalze, d.h. als Aminosäurehydrochloride) eingesetzt werden. Für den Fall, dass die freien Aminosäuren eingesetzt werden, erfolgt die Einstellung des gewünschten pH-Wertes durch Zugabe einer geeigneten physiologisch verträglichen Puffersubstanz, wie z.B. einer organischen oder anorganischen Säure wie Citronensäure und Phosphorsäure, Schwefelsäure, Essigsäure, Ameisensäure oder deren Salze. Bevorzugt sind Citrate und Phosphate, mit denen besonders stabile Lyophilisate erhalten werden.

Als Aminosäuren bevorzugt sind Arginin, Lysin oder Ornithin. Daneben können auch saure Aminosäuren, wie z.B. Glutaminsäure und Asparaginsäure, oder neutrale Aminosäuren, wie z.B. Isoleucin, Leucin und Alanin, bzw. aromatische Aminosäuren, wie z.B. Phenylalanin, Tyrosin oder Tryptophan, Verwendung finden. Der Aminosäuregehalt in der erfindungsgemäßen Zubereitung beträgt 1 bis 100 mg/ml, bevorzugt 1 bis 50 mg/ml, besonders bevorzugt sind 3-30 mg/ml (jeweils bezogen auf die rekonstituierte Lösung).

Als Tenside einsetzbar sind alle in pharmazeutischen Zubereitungen üblicherweise verwendeten Tenside, vorzugsweise Polysorbate und Polyoxyethylen-Polyoxypropylen-Polymere. Besonders bevorzugt sind Polyoxyethylen(20)-sorbitanmonolaurat und Polyoxyethylen(20)-sorbitanmonooleat. Erfindungsgemäß ist in der Zubereitung 0,001 bis 1 Gew.-%, bevorzugt 0,005 bis 0,1 Gew.-% und besonders bevorzugt cirka 0,01 Gew.-% enthalten (jeweils bezogen auf die rekonstituierte Lösung).

Soweit die erfindungsgemäße Zubereitung Puffer enthalten sind, können hierfür grundsätzlich alle physiologisch verträglichen Substanzen eingesetzt werden, die zur Einstellung des gewünschten pH-Wertes geeignet sind. Die Menge an Puffersubstanz wird dabei so gewählt, dass nach Rekonstitution der lyophilisierten Zubereitung, beispielweise mit Wasser für Injektionszwecke, die erhaltene wässrige Lösung eine Pufferkonzentration von 5 mmol/l bis 20 mmol/l, vorzugsweise von cirka 10 mmol/l aufweist. Als Puffer bevorzugt sind Citratpuffer oder Phosphatpuffer. Geeignete Phosphatpuffer sind Lösungen der Mono- und/oder Di-Natrium- und Kaliumsalze der Posphorsäure, wie Dinatriumhydrogenphosphat oder Kaliumdihydrogenphosphat, sowie Mischungen der Natrium- und Kaliumsalze, wie beispielsweise Mischungen aus Dinatriumhydrogenphosphat und Kaliumdihydrogenphosphat.

Soweit durch die osmotischen Eigenschaften des Antikörpers und die zur Stabilisierung eingesetzten Hilfsstoffe die rekonstituierte Lösung nicht bereits isotonisch ist, kann weiterhin ein Isotonisierungsmittel, bevorzugt ein physiologisch verträgliches Salz, wie beispielsweise Natrium- oder Kaliumchlorid, oder ein physiologisch verträgliches Polyol, wie beispielsweise Glucose oder Glycerin, in einer zur Isotonisierung erforderlichen Konzentration enthalten sein.

Darüber hinaus können die erfindungsgemäßen Lyophilisate weitere physiologisch verträgliche Hilfsstoffe, wie z.B. Antioxidantien wie Ascorbinsäure oder Glutathion, Konservierungsmittel wie Phenol, m-Cresol, Methyl- oder Propylparaben, Chlorbutanol, Thiomersal oder Benzalkoniumchlorid, Polyethylenglykole (PEG) wie PEG 3000, 3350, 4000 oder 6000 oder Cyclodextrine wie Hydroxypropyl-β-cyclodextrin, Sulfobutylethyl-β-cyclodextrin oder γ-Cyclodextrin, enthalten.

Die erfindungsgemäße Zubereitung kann hergestellt werden, indem man eine wässrige Zubereitung, enthaltend Cetuximab oder EMD 72000 als Wirkstoff und einen Zucker oder Aminozucker, eine Aminosäure und ein Tensid als Zusatzstoffe sowie gegebenenfalls weitere pharmazeutische Hilfsstoffe, herstellt und die Lösung anschließend lyophilisiert.

Die wässrige Zubereitung kann hergestellt werden, indem man einer Cetuximab bzw. EMD 72000 enthaltenden Lösung die genannten Hilfsstoffe zugefügt werden. Zweckmäßigerweise wird hierzu einer Lösung mit einer definierten Konzentration an Cetuximab bzw. EMD 72000, wie sie bei dessen Herstellung gewonnen wird, mit definierten Volumina an Stammlösungen, die die genannten weiteren Hilfsstoffe in definierter Konzentration enthalten, versetzt und gegebenenfalls mit Wasser auf die vorberechnete Konzentration verdünnt. Alternativ können die Hilfsstoffe der Cetuximab enthaltenden Ausgangslösung auch als Feststoffe zugesetzt werden. Liegt Cetuximab bzw. EMD 72000 als Feststoff, beispielsweise als Lyophilisat, vor, kann die erfindungsgemäße Zubereitung hergestellt werden, indem jeweilige Antikörper zunächst in Wasser oder einer einen oder mehrere der weiteren Hilfsstoffe enthaltenden wässrigen Lösung gelöst und anschließend mit den jeweils erforderlichen Mengen an die weiteren Hilfsstoffe enthaltenden Stammlösungen, mit den weiteren Hilfsstoffen in fester Form und/oder Wasser versetzt werden. Zweckmäßigerweise kann Cetuximab bzw. EMD 72000 auch direkt in einer alle weiteren Hilfsstoffe enthaltenden Lösung gelöst werden. Vorteilhaft kann einer oder mehrere der in der erfindungsgemäßen Zubereitung enthaltenen Hilfsstoffe bereits während oder zum Schluss des Herstellungsverfahrens des jeweiligen EGFR-Antikörpers zugegeben werden. Bevorzugt kann dies dadurch erfolgen, indem Cetuximab bzw. EMD 72000 im letzten Schritt der nach seiner Herstellung erfolgenden Aufreinigung direkt in einer einen, mehrere oder alle weiteren Hilfsstoffe enthaltenden wässrigen Lösung gelöst wird. Dann müssen zur Herstellung der Zubereitung die jeweiligen weiteren Inhaltsstoff/e nur noch in jeweils geringerer Menge und/oder gar nicht zugesetzt werden. Besonders bevorzugt ist, wenn der jeweilige Inhaltsstoff im letzten Schritt der nach seiner Herstellung erfolgenden Aufreinigung direkt in einer alle weiteren Hilfsstoffe enthaltenden wässrigen Lösung gelöst wird, so dass direkt die zu lyophilisierende Lösung erhalten wird.
Die den jeweiligen Antikörper sowie die Hilfsstoffe enthaltende Lösung wird auf einen pH-Wert von 5 bis 8 eingestellt, sterilfiltriert und gefriergetrocknet.

Die Beispiele, ohne darauf beschränkt zu sein, erläutern die Erfindung.

Soweit 10 mmol/l Natrium- bzw. Kaliumphosphatpuffer pH 7,2 eingesetzt wurde, enthielt dieser 2,07 g/l Dinatriumhydrogenphosphat-7-hydrat und 0,31 g/l Natriumdihydrogenphosphat-Monohydrat bzw. 1,220 g/l Dikaliumhydrogenphosphat und 0,4050 g/l Kaliumdihydrogenphosphat.

### Beispiel 1

Lyophilisat aus wässriger Lösung enthaltend:
10 mg/ml EMD 72000
10 mmol/l Kaliumphosphatpuffer pH 7,2
17 mmol/l Arginin
3 Gew.-% Saccharose
0,01 Gew.-% Polyoxyethylen(20)-sorbitanmonolaurat
0,4 Gew.-% PEG 6000

Die Herstellung erfolgte durch Mischung definierter Volumina von die jeweiligen Hilfsstoffe in definierter Konzentration enthaltenden wässrigen Lösungen. Folgende Lösungen wurden verwendet:
Lösung A (Wirkstofflösung) enthaltend:
20 mg/ml EMD 72000
10 mmol/l Kaliumphosphatpuffer pH 7,2

Lösung B (Puffer/Salz-Lösung):
10 mmol/l Kaliumphosphatpuffer pH 7,2
6 Gew.-% Saccharose
0,02 Gew.-% Polyoxyethylen(20)-sorbitanmonolaurat
34 mmol/l Arginin
0,8 Gew.-% Polyethylenglykol 6000

Zur Herstellung der erfindungsgemäßen Zubereitung wurden gleiche Volumina Lösung A und Lösung B miteinander vereinigt.

Die zubereitete Lösung wurde vor der Abfüllung sterilfiltriert. Die Vials wurden mit je 2 ml Lösung befüllt. Anschließend wurden die Vials mit Stopfen partiell verschlossen und lyophilisiert. Nach erfolgter Gefriertrocknung wurden die Vials verschlossen und gebördelt.

### Beispiel 2

Lyophilisat aus wässriger Lösung enthaltend:
10 mg/ml EMD 72000
10 mmol/l Kaliumphosphatpuffer pH 7,2
14 mmol/l Arginin
3 Gew.-% Saccharose
0,01 Gew.-% Polyoxyethylen(20)-sorbitanmonolaurat

Die Herstellung erfolgte durch Mischung definierter Volumina von die jeweiligen Hilfsstoffe in definierter Konzentration enthaltenden wässrigen Lösungen. Folgende Lösungen wurden verwendet:
Lösung A (Wirkstofflösung) enthaltend:
20 mg/ml EMD 72000
10 mmol/l Kaliumphosphatpuffer pH 7,2

Lösung B (Puffer/Salz-Lösung):
10 mmol/l Kaliumphosphatpuffer pH 7,2
6 Gew.-% Saccharose
0,02 Gew.-% Polyoxyethylen(20)-sorbitanmonolaurat
34 mmol/l Arginin

Zur Herstellung der erfindungsgemäßen Zubereitung wurden gleiche Volumina Lösung A und Lösung B miteinander vereinigt.

Die zubereitete Lösung wurde vor der Abfüllung sterilfiltriert. Die Vials wurden mit je 20 ml Lösung befüllt. Anschließend wurden die Vials mit Stopfen partiell verschlossen und lyophilisiert. Nach erfolgter Gefriertrocknung wurden die Vials verschlossen und gebördelt.

### Beispiel 3

Lyophilisat aus wässriger Lösung enthaltend:
15 mg/ml Cetuximab
5 mmol/l Citrat
100 mmol/l Arginin
4 Gew.-% Mannitol
0,01 Gew.-% Polyoxyethylen(20)-sorbitanmonooleat

Die Herstellung erfolgte durch Mischung definierter Volumina von die jeweiligen Hilfsstoffe in definierter Konzentration enthaltenden wässrigen Lösungen. Folgende Lösungen wurden verwendet;
Lösung A (Wirkstofflösung) enthaltend:
19 mg/ml Cetuximab
5 mmol/l Citrat
127 mmol/l Arginin
0,01 Gew.-% Polyoxyethylen(20)-sorbitanmonooleat

Lösung B (Puffer/Salz-Lösung):
5 mmol/l Citrat
19.05 Gew.-% Mannitol
0,01 Gew.-% Polyoxyethylen(20)-sorbitammonooleat

Zur Herstellung der erfindungsgemäßen Zubereitung wurden 7.9 ml Lösung A und 2.1 ml Lösung B miteinander vereinigt.

Die zubereitete Lösung wurde vor der Abfüllung mit einem Sterilfilter filtriert. Die Vials wurden mit einer Pipette mit je 2 ml Lösung befüllt. Anschließend wurden die Vials mit Stopfen partiell verschlossen und lyophilisiert. Nach erfolgter Gefriertrocknung wurden die Vials verschlossen und gebördelt.

### Beispiel 4

Lyophilisat aus wässriger Lösung enthaltend:
15 mg/ml Cetuximab
5 mmol/l Citrat
100 mmol/l Arginin
1,5 Gew.-% Saccharose
0,01 Gew.-% Polyoxyethylen(20)-sorbitanmonooleat

Die Herstellung erfolgte durch Mischung definierter Volumina von die jeweiligen Hilfsstoffe in definierter Konzentration enthaltenden wässrigen Lösungen. Folgende Lösungen wurden verwendet:
Lösung A (Wirkstofflösung) enthaltend:
19 mg/ml Cetuximab
5 mmol/l Citrat
127 mmol/l Arginin
0,01 Gew.-% Polyoxyethylen(20)-sorbitanmonooleat

Lösung B (Puffer/Salz-Lösung):
5 mmol/l Citrat
7,1% Saccharose
0,01 Gew.-% Polyoxyethylen(20)-sorbitanmonooleat

Zur Herstellung der erfindungsgemäßen Zubereitung wurden 7.9 ml Lösung A und 2.1 ml Lösung B miteinander vereinigt.

Die zubereitete Lösung wurde vor der Abfüllung mit einem Sterilfilter filtriert. Die Vials wurden mit einer Pipette mit je 2 ml Lösung befüllt. Anschließend wurden die Vials mit Stopfen partiell verschlossen und lyophilisiert. Nach erfolgter Gefriertrocknung wurden die Vials verschlossen und gebördelt.

### Beispiel 5

Lyophilisat aus wässriger Lösung enthaltend:
15 mg/ml Cetuximab
10 mmol/l Kaliumphosphatpuffer pH 7,2
14 mmol/l L-Argininhydrochlorid
88 mmol/l Saccharose
0,01 Gew.-% Polyoxyethylen(20)-sorbitanmonolaurat
ad pH 7,5 mit Phosphorsäure einstellen

Die Herstellung erfolgte durch Mischung definierter Volumina von die jeweiligen Hilfsstoffe in definierter Konzentration enthaltenden wässrigen Lösungen. Folgende Lösungen wurden verwendet:
Lösung A (Wirkstofflösung) enthaltend:
25 mg/ml Cetuximab
10 mmol/l Kaliumphosphatpuffer pH 7,2
Wasser für Injektionszwecke

Lösung B (Puffer/Salz-Lösung):
10 mmol/l Kaliumphosphatpuffer pH 7,2
35,6 mmol/l L-Argininhydrochlorid
219 mmol/l Saccharose
0,025 Gew.-% Polyoxyethylen(20)-sorbitanmonolaurat
ad pH 7,5 mit Phosphorsäure einstellen

Zur Herstellung der erfindungsgemäßen Zubereitung wurden 6 ml Lösung A und 4 ml Lösung B miteinander vereinigt.

Die zubereitete Lösung wurde vor der Abfüllung mit einem Sterilfilter filtriert. Die Vials wurden mit einer Pipette mit je 2 ml Lösung befüllt. Anschließend wurden die Vials mit Stopfen partiell verschlossen und lyophilisiert. Nach erfolgter Gefriertrocknung wurden die Vials verschlossen und gebördelt.

### Beispiel 6 (Vergleichszubereitung 1)

Wässrige Lösung enthaltend:
5 mg/ml Cetuximab
10 mmol/l Natriumphosphatpuffer pH 7,2
145 mmol/l Natriumchlorid

Die Herstellung erfolgte durch Mischung definierter Volumina von die jeweiligen Hilfsstoffe in definierter Konzentration enthaltenden wässrigen Lösungen. Folgende Lösungen wurden verwendet:
Lösung A (Wirkstofflösung) enthaltend:
10 mg/ml Cetuximab
10 mmol/l Natriumphosphatpuffer pH 7,2
145 mmol/l Natriumchlorid.

(Die Lösung wurde erhalten, indem der Wirkstoff im letzten Schritt der nach dessen Herstellung erfolgenden chromatographischen Wirkstoffaufreinigung mit Lösung B von der Säule eluiert wurde.)

Lösung B (Puffer/Salz-Lösung):
entspricht Lösung A, enthält jedoch kein Wirkstoff.

Zur Herstellung der Vergleichszubereitung wurden jeweils 10 ml der Lösungen A und B miteinander vereinigt.

### Beispiel 7 (Vergleichszubereitung 2)

Wässrige Lösung enthaltend:
5 mg/ml Cetuximab
10 mmol/l Natriumphosphatpuffer pH 7,2
45 mmol/l Natriumchlorid
0,01 Gew.-% Polyoxyethylen(20)-sorbitanmonooleat.

Die Herstellung erfolgte durch Mischung definierter Volumina von die jeweiligen Hilfsstoffe in definierter Konzentration enthaltenden wässrigen Lösungen. Folgende Lösungen wurden verwendet:
Lösung A (Wirkstofflösung) enthaltend:
9,7 mg/ml Cetuximab
10 mmol/l Natriumphosphatpuffer pH 7,2
145 mmol/l Natriumchlorid.
(Die Lösung wurde erhalten, indem der Wirkstoff im letzten Schritt der nach dessen Herstellung erfolgenden chromatographischen Wirkstoffaufreinigung mit Lösung B von der Säule eluiert wurde.)
Lösung B (Puffer/Salz-Lösung):
entspricht Lösung A, enthält jedoch kein Wirkstoff.

Lösung C (Polyoxyethylensorbitan-Fettsäureester-Lösung):
entspricht Lösung B, enthält jedoch zusätzlich 1 Gew.-%
Polyoxyethylen(20)-sorbitanmonooleat.

Zur Herstellung der erfindungsgemäßen Zubereitung wurden 10 ml Lösung A, 9,8 ml Lösung B und 0,2 ml Lösung C miteinander vereinigt.

Die zubereitete Lösung wurde vor der Abfüllung mit einem Sterilfilter filtriert. Die Vials wurden mit einer Pipette mit je 2 ml Lösung befüllt. Anschließend wurden die Vials mit Stopfen verschlossen und gebördelt.

### Untersuchungen zur Stabilität der Zubereitungen

Die Stabilität der erfindungsgemäßen Zubereitungen der Beispiele 1 und 2 wurde in Stresstests geprüft. Hierzu wurden die hergestellten Lyophilisate bei 40°C und einer relativen Luftfeuchtigkeit (r.F.) von 75% eingelagert. Die Zubereitungen wurden zu bestimmten Zeiten ausgelagert und mit geeigneten analytischen Methoden untersucht. Mögliche Instabilitäten äußern sich bei Antikörpern vornehmlich in der Bildung von Aggregaten als auch in der Bildung von Abbauprodukten. Abbauprodukte werden bevorzugt durch Gelelektrophoresen (Natrium-dodecylsulfat-Polyacrylamidgelelektrophorese (SDS-PAGE) und Isoelektrische Fokussierung (IEF)) detektiert, während visuelle Inspektion und Trübungsmessungen zum Nachweis von sichtbaren Aggregaten und die Größenausschlusschromatographie (HPLC-SEC) zum Nachweis von löslichen Aggregaten dienen. Der ebenfalls zur Evaluierung der Zubereitungen herangezogene ELISA-Test (Enzyme Linked Immunosorbent Assay) dient zur Überprüfung der Integrität und der Bindungsfähigkeit am Rezeptor.

Die Ergebnisse der Tabellen 1 und 2 belegen klar die Qualität und die Stabilität der hergestellten Zubereitungen auch bei erhöhten Lagerungstemperaturen und erhöhter relativer Luftfeuchtigkeit (40°C / 75% r.F.).

**Tabelle 1: Stabilität der Zubereitung gemäß Beispiel 1**

| Lagerdauer (Monate) | pH-Wert | ELISA (rel. Titer) | SDS-PAGE [% IG-Monomer] | | SE-HPLC |
|---|---|---|---|---|---|
| | | | nichtreduzierend | reduzierend | |
| Temperatur 2 - 8°C | | | | | |
| 0 | 7.11 | 1.00 | 100 | 100 | 100 |
| 6 | 7.14 | 1.01 | 99.3 | 100 | 99.6 |
| 12 | 7.15 | 1.04 | 99.9 | 99.1 | 99.7 |
| 24 | 7.16 | 1.03 | 100 | 100 | 100 |

| Temperatur 25°C, 60% rel. Feuchtigkeit | | | | | |
|---|---|---|---|---|---|
| 6 | 7.17 | 1.03 | 99.1 | 99.0 | 99.5 |
| 12 | 7.15 | 1.01 | 98.7 | 99.1 | 99.6 |
| 24 | 7.16 | 1.03 | 100 | 100 | 99.5 |

| Temperatur 40°C, 75% rel. Feuchtigkeit | | | | | |
|---|---|---|---|---|---|
| 6 | 7.17 | 1.10 | 98.8 | 98.2 | 99.3 |
| 12 | 7.15 | n.d. | n.d. | n.d. | n.d. |
| 24 | 7.16 | 0.98 | 100 | 100 | 99.3 |

**Tabelle 2: Stabilität der Zubereitung gem. Beispiel 2**

| Lagerdauer (Wochen) | pH-Wert | ELISA (rel. Titer) | SDS-PAGE [% IG-Monomer] | | SE-HPLC |
|---|---|---|---|---|---|
| | | | nichtreduzierend | reduzierend | |
| Temperatur 2 - 8°C | | | | | |
| 0 | 7.20 | 0.97 | 100 | 100 | 99.33 |
| 13 | 7.23 | 1.00 | n.d. | n.d. | 99.24 |
| 26 | 7.21 | 1.02 | 99.47 | 99.57 | 99.27 |

| Temperatur 25°C, 60% rel. Feuchtigkeit | | | | | |
|---|---|---|---|---|---|
| 13 | 7.24 | 1.05 | n.d. | n.d. | 99.22 |
| 26 | 7.24 | 0.97 | 99.47 | 99.64 | 99.21 |

| Temperatur 40°C, 75% rel. Feuchtigkeit | | | | | |
|---|---|---|---|---|---|
| 13 | 7.23 | 1.04 | n.d. | n.d. | 99.08 |
| 26 | 7.25 | 0.97 | 99.61 | 99.62 | 98.85 |

Die Stabilität der erfindungsgemäßen Zubereitungen der Beispiele 3 bis 7 wurde ebenfalls in Stresstests überprüft. Hierzu wurden Vials enthaltend die Lösung gemäß Beispiel 3-5 sowie zu Vergleichszwecken Vials enthaltend Lösung gemäß Beispiel 6-7 bei 25°C und 60% relativer Luftfeuchtigkeit (r.F) und 40°C und 75% r.F. eingelagert. Vor Einlagerung sowie nach definierten Lagerzeiten wurden jeweils 3 Vials visuell bei direkter Anstrahlung mit einer Kaltlichtquelle beurteilt und die Absorption der Lösungen bei 350 und 550 nm bestimmt, die ein Maß für die Trübung darstellt. Weiterhin wurden jeweils 3 Vials entnommen und hinsichtlich des Gehaltes an Cetuximab und Zersetzungsprodukten mittels HPLC-Gelfiltration untersucht.

Die HPLC-chromatographischen Untersuchungen erfolgte mit Gradienten aus Acetonitril / Wasser 95/5 (VN) (B) und Pufferlösung pH 2,5 / Acetonitril 95/5 (V/V) (A) als Eluenten. Säule: LiChroCHART^{®} HPLC-Kartusche 125-2; Superspher^{®} 60 RP-select B, Fluss: 0,3 ml/min, Detektion bei 210 nm.

Die Ergebnisse der Stabilitätsuntersuchungen sind in Tabelle 3 dargestellt.

**Tabelle 3**

| Prü- fungslösung | Lagerung bei 40°C / 75% r.F [Wochen] | Cetuximab [%] | Aggregate [%] | Zersetzungsprodukte [%] | Trübung bei λ= 350 nm | Trübung bei λ= 550 nm | visuelle Beurteilung |
|---|---|---|---|---|---|---|---|
| Bsp. 3 | 0 | 99,30 | 0,20 | 0,51 | 0,0211 | 0,0055 | klar |
| Bsp. 3 | 4 | 99,04 | 0,62 | 0,34 | 0,0214 | 0,0029 | klar |
| Bsp.3 | 8 | 98,54 | 1,12 | 0,34 | 0,0224 | 0,0020 | klar |
| Bsp. 3 | 13 | 98,43 | 1,18 | 0,40 | 0,0246 | 0,0019 | klar |
| | | | | | | | |
| Bsp.4 | 0 | 99,33 | 0,19 | 0,48 | 0,0198 | 0,0045 | klar |
| Bsp. 4 | 4 | 99,11 | 0,40 | 0,50 | 0,0174 | 0,0025 | klar |
| Bsp. 4 | 8 | 99,19 | 0,44 | 0,38 | 0,0181 | 0,0021 | klar |
| Bsp. 4 | 13 | 99,20 | 0,45 | 0,36 | 0,0172 | 0,0014 | klar |
| | | | | | | | |
| Bsp. 5 | 0 | 99,58 | 0,19 | 0,04 | 0,0195 | 0,0050 | klar |
| Bsp. 5 | 4 | 99,51 | 0,24 | 0,25 | 0,0165 | 0,0028 | klar |
| Bsp. 5 | 8 | 99,56 | 0,22 | 0,22 | 0,0156 | 0,0023 | klar |
| Bsp. 5 | 13 | 99,52 | 0,28 | 0,20 | 0,0187 | 0,0045 | klar |
| | | | | | | | |
| Bsp. 6 | 0 | 99,69 | 0,62 | 0,15 | 0,0130 | 0,0021 | klar |
| Bsp. 6 | 4 | 92,00 | 0,84 | 7,38 | 0,0232 | 0,0047 | trüb |
| Bsp. 6 | 8 | 89,94 | 1,39 | 8,68 | 0,0338 | 0,0044 | trüb |
| Bsp.6 | 13 | 86,66 | 3,26 | 10,11 | 0,0403 | 0,0061 | trüb |
| | | | | | | | |
| Bsp.7 | 0 | 99,72 | 0,17 | 0,11 | 0,0128 | 0,0016 | klar |
| Bsp. 7 | 4 | 98,60 | 0,56 | 0,84 | 0,0200 | 0,0022 | klar |
| Bsp. 7 | 8 | 96,49 | 2,21 | 1,32 | 0,0280 | 0,0033 | klar |
| Bsp.7 | 13 | 86,46 | 4,84 | 8,73 | 0,0382 | 0,0036 | trüb |

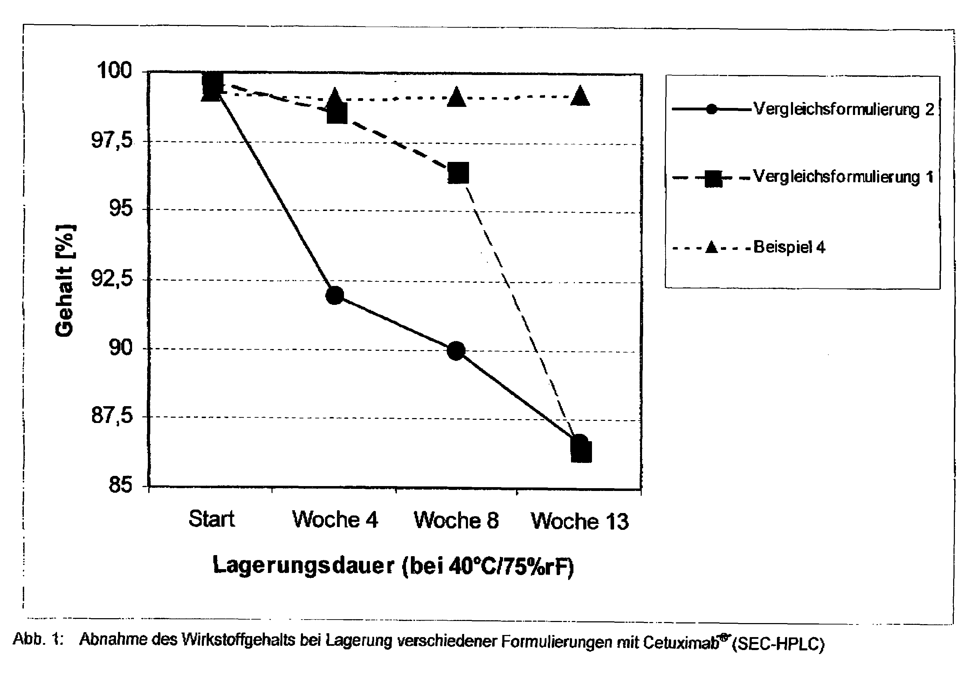

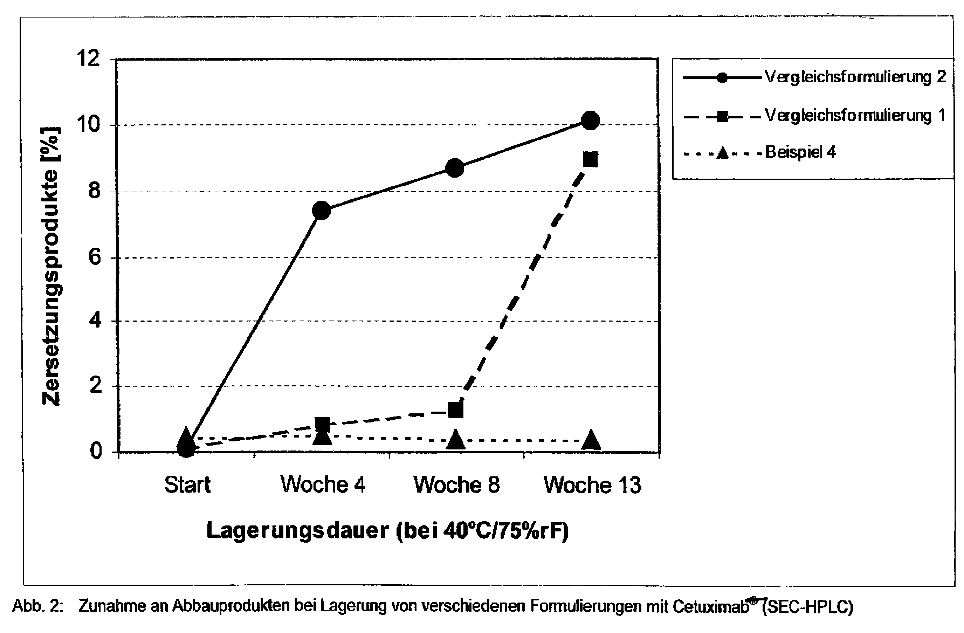

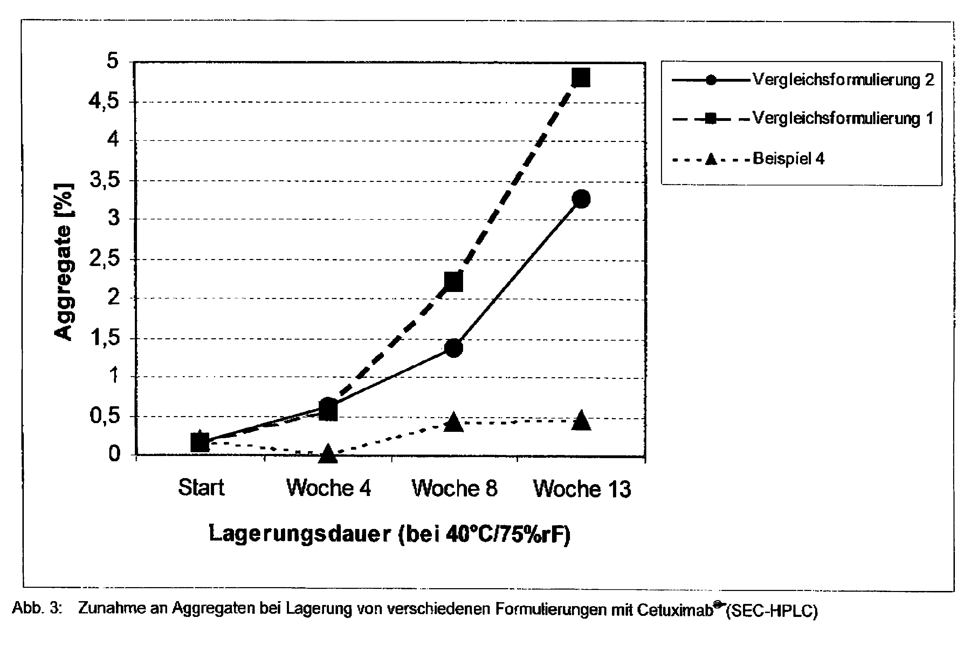

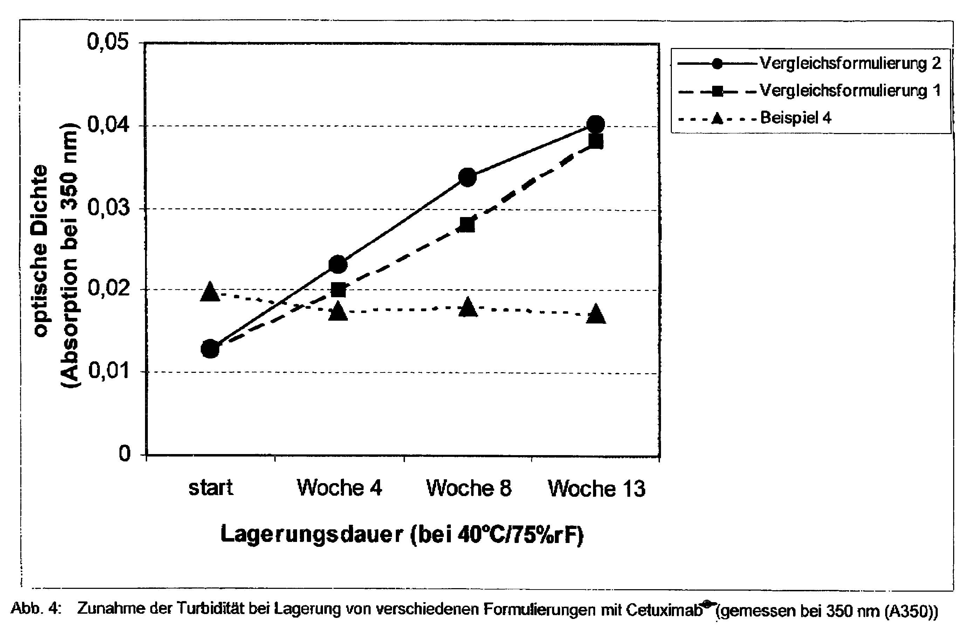

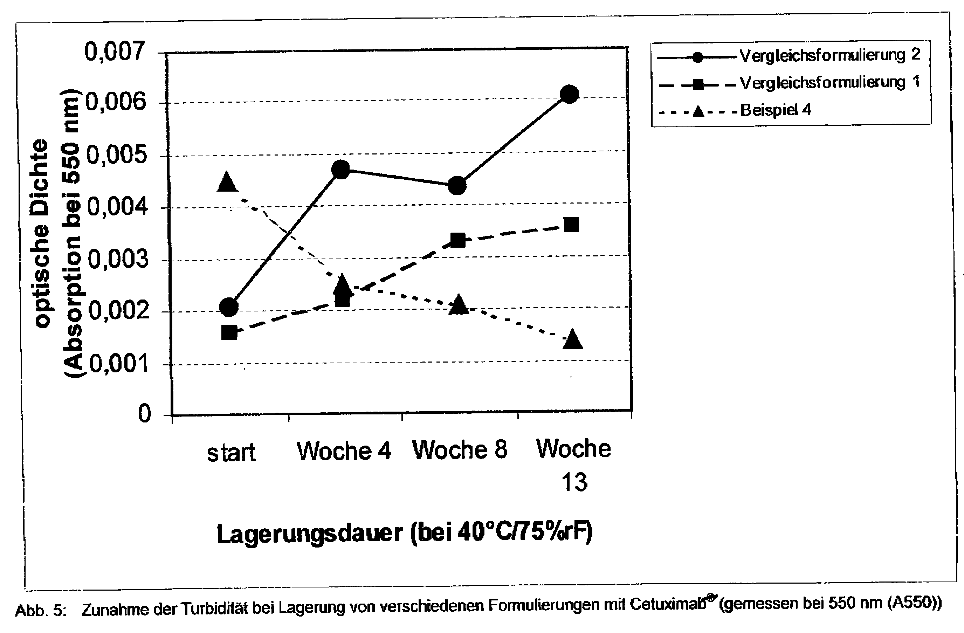

Abbildungen 1 bis 5 zeigen weiterhin im Vergleich die Ergebnisse verschiedener Stabilitätsuntersuchungen von der erfindungsgemäßen Zubereitung gemäß Beispiel 4 gegenüber den Vergleichsformulierungen 1 und 2 nach definierten Lagerzeiten bei 40°C und 75% r.F.. Die gefriergetrocknete Zubereitung nach Beispiel 4 wurde jeweils vor Durchführung der Analytik mit Wasser für Injektionszwecke zu einer wässrigen Lösung mit der dreifachen Menge Wasser im Vergleich zu der Ausgangslösung, die zur Herstellung der lyophilisierten Zubereitung durch Gefriertrocknung verwendet wurde, rekonstituiert.

Abbildung 1 zeigt die Abnahme des Wirkstoffgehalts in der Vergleichsformulierungen 1 und 2 gegenüber der erfindungsgemäßen Zubereitung gemäß Beispiel 4, gemessen als Gehalt an Monomer in der HPLC-SEC.

Abbildung 2 zeigt die Zunahme an Abbauprodukten in den Vergleichsformulierungen 1 und 2 gegenüber der erfindungsgemäßen Zubereitung gemäß Beispiel 4, gemessen in der HPLC-SEC.

Abbildung 3 zeigt die Zunahme an Aggregaten in den Vergleichsformulierungen 1 und 2 gegenüber der erfindungsgemäßen Zubereitung gemäß Beispiel 4, gemessen in der HPLC-SEC.

Abbildung 4 zeigt die Zunahme der optischen Dichte bei λ = 350 nm in den Vergleichsformulierungen 1 und 2 gegenüber der erfindungsgemäßen Zubereitung gemäß Beispiel 4.

Abbildung 5 zeigt die Zunahme der optischen Dichte bei λ = 550 nm in den Vergleichsformulierungen 1 und 2 gegenüber der erfindungsgemäßen Zubereitung gemäß Beispiel 4.

Die Ergebnisse zeigen deutlich, dass die erfindungsgemäße Zusammensetzungen gegenüber den flüssigen Vergleichslösungen eine deutlich erhöhte Stabilität aufweist.

## Patentansprüche

1. Lyophilisierte pharmazeutische Zubereitung von mono- oder polyklonalen Antikörpern, enthaltend einen Zucker oder einen Aminozucker, eine Aminosäure und ein Tensid, **dadurch gekennzeichnet, dass** der Antikörper Cetuximab oder Matuzumab ist

2. Lyophilisierte pharmazeutische Zubereitung gemäß Anspruch 1, bestehend aus einem Antikörper, einem Zucker oder Aminozucker, einer Aminosäure, einem Puffer und einem Tensid

3. Lyophilisierte pharmazeutische Zubereitung gemäß einem oder mehreren der Anspruch 1 und/oder 2, **dadurch gekennzeichnet, dass** der Zucker ein Mono-, Di- oder Trisaccharid, vorzugsweise Saccharose, Maltose oder Trehalose ist

4. Lyophilisierte pharmazeutische Zubereitung gemäß einem oder mehreren der Ansprüche 1 bis 3, **dadurch gekennzeichnet, dass** der Aminozucker Glucosamin, N-Methylglucosamin, Galactosamin oder Neuraminsäure ist

5. Lyophilisierte pharmazeutische Zubereitung gemäß einem oder mehreren der Ansprüche 1 bis 4, **dadurch gekennzeichnet, dass** die Aminosäure Arginin, Lysin, oder Ornithin ist.

6. Lyophilisierte pharmazeutische Zubereitung gemäß einem oder mehreren der Ansprüche 1 bis 5, **dadurch gekennzeichnet, dass** das Tensid ein Polysorbat oder ein Polyoxyethylen-Polyoxypropylen-Polymer ist

7. Lyophilisierte pharmazeutische Zubereitung gemäß Anspruch 6, **dadurch gekennzeichnet, dass** das Tensid Polyoxyethylensorbitan-Fettsäureester Polyoxyethylen(20)-sorbitanmonooleat oder Polyoxyethylen(20)-sorbitanmonolaurat ist

8. Lyophilisierte pharmazeutische Zubereitung gemäß einem oder mehreren der Ansprüche 1 bis 7, **dadurch gekennzeichnet, dass** weiterhin ein Isotonisierungsmittel in einer zur Isotonisierung erforderlichen Konzentration enthalten ist

9. Lyophilisierte pharmazeutische Zubereitung gemäß Anspruch 9, **dadurch gekennzeichnet, dass** Natriumchlorid als Isotonisierungsmittel enthalten ist

10. Wässrige pharmazeutische Zubereitung von mono- oder polyklonalen Antikörpern erhältlich durch Rekonstitution des Lyophilisats nach einem oder mehreren der Ansprüche 1 bis 9 mit einem wässrigen Lösungsmittel

11. Wässrige pharmazeutische Zubereitung nach Anspruch 10, **dadurch gekennzeichnet, dass** die Lösung einen pH-Wert von 5 - 8, vorzugsweise von 6 - 7,4 aufweist

12. Wässrige pharmazeutische Zubereitung nach Anspruch 11, **dadurch gekennzeichnet, dass** die Lösung einen pH-Wert von cirka 7,2 aufweist

13. Verfahren zur Herstellung einer lyophilisierten pharmazeutischen Zubereitung gemäß einem oder mehreren der Ansprüche 1 bis 9, **dadurch gekennzeichnet, dass** man eine wässrige Zubereitung, enthaltend Cetuximab oder Matuzumab als Wirkstoff und einen Zucker oder Aminozucker, eine Aminosäure und ein Tensid als Zusatzstoffe sowie gegebenenfalls weitere pharmazeutische Hilfsstoffe, herstellt und die Lösung anschließend lyophilisiert

## Claims

1. Lyophilised pharmaceutical preparation of mono- or polyclonal antibodies comprising a sugar or an amino sugar, an amino acid and a surfactant, **characterised in that** the antibody is cetuximab or matuzumab.

2. Lyophilised pharmaceutical preparation according to Claim 1, consisting of an antibody, a sugar or amino sugar, an amino acid, a buffer and a surfactant.

3. Lyophilised pharmaceutical preparation according to one or more of Claims 1 and/or 2, **characterised in that** the sugar is a mono-, di- or trisaccharide, preferably sucrose, maltose or trehalose.

4. Lyophilised pharmaceutical preparation according to one or more of Claims 1 to 3, **characterised in that** the amino sugar is glucosamine, N-methylglucosamine, galactosamine or neuraminic acid.

5. Lyophilised pharmaceutical preparation according to one or more of Claims 1 to 4, **characterised in that** the amino acid is arginine, lysine or ornithine.

6. Lyophilised pharmaceutical preparation according to one or more of Claims 1 to 5, **characterised in that** the surfactant is a polysorbate or a polyoxyethylene-polyoxypropylene polymer.

7. Lyophilised pharmaceutical preparation according to Claim 6, **characterised in that** the surfactant is the polyoxyethylene sorbitan fatty acid ester, polyoxyethylene (20) sorbitan monooleate or polyoxyethylene (20) sorbitan monolaurate.

8. Lyophilised pharmaceutical preparation according to one or more of Claims 1 to 7, **characterised in that** an isotonic agent is furthermore present in a concentration necessary for establishing isotonicity.

9. Lyophilised pharmaceutical preparation according to Claim 8, **characterised in that** sodium chloride is present as isotonic agent.

10. Aqueous pharmaceutical preparation of mono- or polyclonal antibodies obtainable by reconstituting the lyophilisate according to one or more of Claims 1 to 9 with an aqueous solvent.

11. Aqueous pharmaceutical preparation according to Claim 10, **characterised in that** the solution has a pH of 5 - 8, preferably 6 - 7.4.

12. Aqueous pharmaceutical preparation according to Claim 11, **characterised in that** the solution has a pH of about 7.2.

13. Process for the preparation of a lyophilised pharmaceutical preparation according to one or more of Claims 1 to 9, **characterised in that** an aqueous preparation comprising cetuximab or matuzumab as active compound and a sugar or amino sugar, an amino acid and a surfactant as additives and, if desired, further pharmaceutical auxiliaries is prepared, and the solution is subsequently lyophilised.

## Revendications

1. Préparation pharmaceutique lyophilisée d'anticorps mono- ou polyclonaux comprenant un sucre ou un sucre aminé, un acide aminé et un agent tensioactif, **caractérisée en ce que** l'anticorps est le cetuximab ou le matuzumab.

2. Préparation pharmaceutique lyophilisée selon la revendication 1, constituée d'un anticorps, d'un sucre ou d'un sucre aminé, d'un acide aminé, d'un tampon et d'un agent tensioactif.

3. Préparation pharmaceutique lyophilisée selon l'une ou plusieurs parmi les revendications 1 et/ou 2, **caractérisée en ce que** le sucre est un mono-, di- ou trisaccharide, préférablement le saccharose, le maltose ou le tréhalose.

4. Préparation pharmaceutique lyophilisée selon l'une ou plusieurs parmi les revendications 1 à 3, **caractérisée en ce que** le sucre aminé est la glucosamine, la N-méthylglucosamine, la galactosamine ou l'acide neuraminique.

5. Préparation pharmaceutique lyophilisée selon l'une ou plusieurs parmi les revendications 1 à 4, **caractérisée en ce que** l'acide aminé est l'arginine, la lysine ou l'ornithine.

6. Préparation pharmaceutique lyophilisée selon l'une ou plusieurs parmi les revendications 1 à 5, **caractérisée en ce que** l'agent tensioactif est un polysorbate ou un polymère de polyoxyéthylène-polyoxypropylène.

7. Préparation pharmaceutique lyophilisée selon la revendication 6, **caractérisée en ce que** l'agent tensioactif est l'ester d'acide gras et de sorbitan polyoxyéthyléné, le monooléate de polyoxyéthylène (20) sorbitan ou le monolaurate de polyoxyéthylène (20) sorbitan.

8. Préparation pharmaceutique lyophilisée selon l'une ou plusieurs parmi les revendications 1 à 7, **caractérisée en ce qu'**un agent isotonique est en outre présent en une concentration nécessaire à l'établissement de l'isotonicité.

9. Préparation pharmaceutique lyophilisée selon la revendication 8, **caractérisée en ce que** du chlorure de sodium est présent comme agent isotonique.

10. Préparation pharmaceutique aqueuse d'anticorps mono- ou polyclonaux pouvant être obtenue par reconstitution du lyophilisat selon l'une ou plusieurs parmi les revendications 1 à 9 avec un solvant aqueux.

11. Préparation pharmaceutique aqueuse selon la revendication 10, **caractérisée en ce que** la solution a un pH de 5 - 8, préférablement de 6 - 7,4.

12. Préparation pharmaceutique aqueuse selon la revendication 11, **caractérisée en ce que** la solution a un pH d'environ 7,2.

13. Procédé de préparation d'une préparation pharmaceutique lyophilisée selon l'une ou plusieurs parmi les revendications 1 à 9, **caractérisée en ce que** l'on prépare une préparation aqueuse comprenant du cetuximab ou du matuzumab comme composé actif et un sucre ou un sucre aminé, un acide aminé et un agent tensioactif comme additifs et, si on le souhaite, des agents auxiliaires pharmaceutiques supplémentaires, puis la solution est lyophilisée.
